# EUROPEAN PATENT SPECIFICATION

(11) **EP 3 290 006 B1**
(45) Date of publication and mention of the grant of the patent: **24.01.2024**
(21) Application number: 16186234.7
(22) Date of filing: 30.08.2016
(51) Int. Cl.: A61F 2/66, A61F 2/80, A61F 2/50, A61F 2/60

(54) **PROSTHETIC DEVICE**
PROTHESENVORRICHTUNG
DISPOSITIF PROTHÉTIQUE

(43) Date of publication of application: 07.03.2018
(73) Proprietor: Fillauer Europe AB, 191 27 Sollentuna (SE)
(72) Inventor: RAMIREZ, Christoffer, 181 50 Vallentuna (SE)
(74) Representative: Brann AB

(56) References cited:
- WO-A2-2007/008803
- US-A- 4 911 724
- US-A- 4 994 086
- US-A- 5 509 936
- US-A1- 2002 177 906
- US-A1- 2007 213 840
- US-A1- 2013 030 549

## Description

### FIELD OF THE INVENTION

The present invention relates generally to prosthetic devices, and particularly to prosthetic devices for the user's lower limbs. WO 2007/008803 A2 discloses the features of the preamble of claim 1.

### BACKGROUND AND DESCRIPTION OF THE PRIOR ART

Many individuals have lost a limb for various reasons including war, accident, or disease. In most instances these individuals are not only able to live relatively normal lives, but physically active lives including athletics. Often times, these individuals are aided in their physically active lives by a special athletic prosthetic limb. The objective of prosthesis is to provide an artificial prosthetic limb that simulates the function during athletics of the replaced limb.

It is known to use a prosthetic device on a user's lower limb. Conventional lower limb prosthetic devices, however, suffer from one or more shortcomings as special athletic prosthetic limbs. For example, conventional lower limb prosthetic devices are slow since they typically include slow hinge and dampener components. Conventional lower limb prosthetic devices are also undesirably heavy and large because of the heavy components. In addition, conventional higher limb prosthetic devices also have undesirable characteristics such as being too soft, not stiff enough. Conventional prosthetic devices are also undesirably complex and lack durability for athletics.

It would be desirable, therefore, if an lower limb prosthetic device could be provided that is fast also during athletics. It would also be desirable if such a device could be provided that would reduce the weight and size of the lower limb prosthetic device. It would be further desirable if an lower limb prosthetic device could be provided that would improve characteristics of the upper prosthetic device.

### SUMMARY OF THE INVENTION

According to an aspect, there is provided a lower limb prosthetic device adapted to be connected to an attachment section, configured to attach to the limb, for instance to a socket to receive the limb of an amputee, the latter sometimes also referred to as a "wearer" or "user". The prosthetic device comprises a leaf spring member having a proximal, upper, end. The proximal end can be fixedly and/or adjustably attached to the attachment section. The leaf spring member further comprises a distal, lower, end. The distal end can be attached to a prosthetic foot member.

Herein, this disclosure, the term "lower limb" is intended to include any lower limb of an amputee, i. e. below a waist of an amputee, but is in particular related to a lower limb over the knee of an amputee.

The prosthetic foot member can be a specific prosthetic athletic foot of any suitable conventional type intended for s specific athletic use such as skiing. The prosthetic foot member per se can also be of conventional type and hence the invention is not primarily related to the type of prosthetic foot member per se.

In addition, according to an aspect, the spring member has an original configuration in unloaded condition and is adapted to be compressed when a load is applied to the lower limb prosthetic device and return to the original configurations when the load is removed from the device. In this way, the lower limb prosthetic device of embodiments of the invention can help to propel the user's step, which may be important, or an advantage during athletics.

An advantage with embodiments of the invention is that the problem with slow hinge and dampener components are solved. This may be a considerable problem for a lower limb over the knee of an amputee, wherein a conventional prosthetic device typically comprises an artificial knee including some type of hinge element.

Typically, the leaf spring member comprises an arch and is resilient.

An advantage of the embodiments of the invention is an improved athletic lower limb prosthetic device solving or reducing one or more of the shortcomings of special athletic prosthetic limbs. Herein, the term "athletic device" includes any kind of such device for professional, as well as non-professional use, without any limitation.

Accordingly, it is an advantage of at least some embodiments of the invention claimed herein to provide a lower limb prosthetic device that increases the speed of the device also during athletics. It is also an advantage of the preferred embodiments of the invention claimed herein to provide a lower limb prosthetic device that reduces the weight and size of the prosthetic device.

Additional advantages of the invention will become apparent from the description of the embodiments and accompanying drawings.

### BRIEF DESCRIPTION OF THE DRAWINGS

The presently preferred embodiments of the invention are illustrated in the accompanying drawings, in which like reference numerals represent like parts throughout, and in which:
FIG. 1 is a perspective view of an lower limb prosthetic device with a leaf spring in accordance with an embodiment of the invention;
FIG. 2 is a side view of the lower limb prosthetic device illustrated in FIG. 1.;
FIG. 3 is a perspective view of the lower limb prosthetic device illustrated in use; and
FIG. 4 is a perspective view of the lower limb prosthetic device according to an alternative embodiment.

### DESCRIPTION OF THE PREFERRED EMBODIMENTS OF THE INVENTION

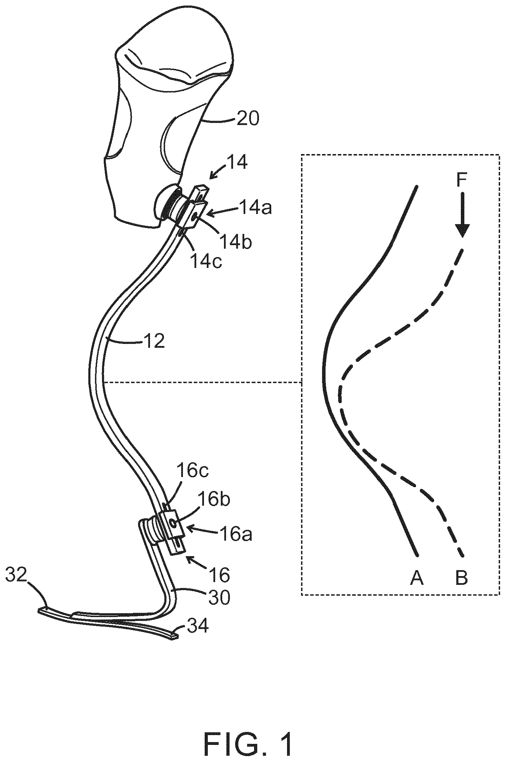

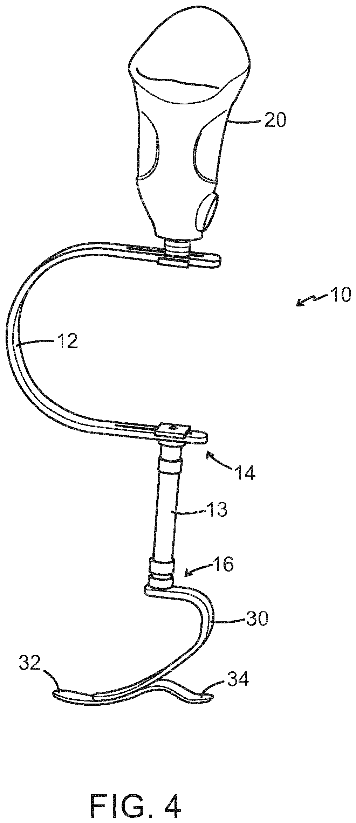

Referring now to the drawings, embodiments of the lower limb prosthetic device with a leaf spring are illustrated by FIGS. 1 through 4. FIGS. 1 to 3 illustrate a first embodiment and FIG. 4 a second alternative embodiment of the lower limb prosthetic device. The lower limb prosthetic devices according to various embodiments are adapted to reduce the weight and size of the prosthetic device. The lower limb prosthetic devices according to various embodiments are further adapted to improve the characteristics of the prosthetic device.

The lower limb prosthetic device 10 according to all embodiments is provided to be used above the location of a user's knee.

Referring now to FIG. 1, a perspective view of an embodiment of the lower limb prosthetic device with a leaf spring is illustrated. As shown in FIG. 1, the lower limb prosthetic device is designated generally by reference numeral 10. The lower limb prosthetic device 10 is adapted to be worn on a user's lower limb (not shown) and is therefore adapted to be connected to, or comprise an attachment section 20 that can include an attachment member, such as a connector or coupler, configured to attach to the limb, sometimes also referred to as "the stump", of an amputee, or a socket to receive the limb, or stump. The attachment member can have a conical connector, pyramidal connector, or the like. The attachment member can be of any suitable conventional type. The attachment section 20 can be, or comprise, a conventional socket.

The lower limb prosthetic device 10 comprises a leaf spring member 12 which has proximal, upper, end 14 and, lower, distal end 16. The distal end 16 of the lower limb prosthetic device 10 can be, or is attached to a foot prosthetic member 30 which has toe end 32 and heel end 34. The foot prosthetic member 30 can be a specific prosthetic athletic foot including an attachment member configured to be attached to the leaf spring 12. The specific prosthetic athletic foot 30 can be of any suitable conventional type intended for a specific athletic use such as skiing.

Still referring to FIG. 1, in the lower limb prosthetic device 10, the proximal end 14 can be fixedly and/or adjustably attached to the attachment section 20, and the distal end 16 can be fixedly and/or adjustably attached to the foot member 30.

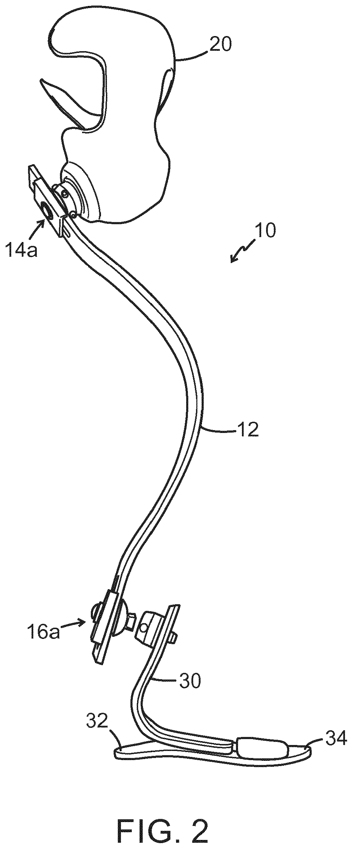

As shown in FIG. 1 and FIG. 2, the lower limb prosthetic device 10 comprises an upper mounting device 14a, leaf spring member 12 and a lower mounting device 16a. The upper 14a, as well as the lower 16a mounting devices are adapted to be adjustably attached by mounting means such as an upper releasable nut and bolt combination 14b, a lower releasable nut and bolt combination 16b, running in a respective upper 14b groove, and lower 16b groove respectively, running in the leaf spring member 12 as shown in FIG. 1 and FIG. 2.

The leaf spring member 12 is of composite type and comprises one or more leaf spring(s), typically one, that comprise(s) an arch and is/are resilient. FIGS. 1-2 illustrate one type of leaf spring and FIG. 4 an alternative type of leaf spring having a, C-formed arc. This latter type of leaf spring being C-formed will be further described in relation to FIG. 4.

In addition, according to an aspect, the leaf spring member 12 has an original configuration in unloaded condition and is adapted to be compressed when a load such as a user's weight provided via the user's limb is applied to the lower limb prosthetic device 10 and return to the original configurations when the load is removed from the lower limb prosthetic device 10. The leaf spring member 12 increases in stiffness when deflected vertically and compressed by the load being part of the weight of the wearer. The energy stored by the leaf spring member 12 can be returned to the user when the user lifts up on the prosthetic foot 30 and hence the load is removed from the prosthetic device 10. This increases the resilient help compared to conventional lower limb prosthetic device using mechanical hinges and hydraulics. In this way, the lower limb prosthetic device 10 of the present invention can help to propel the user's step, which may be important, or an advantage during athletics.

The dotted line A in the inset in FIGS. 1 shows the prosthetic device in an unloaded condition. The dotted line B in the inset shows the lower limb prosthetic device in a loaded condition, storing energy. As shown by reference letters A and B, the leaf spring 12 is vertically displaced in a downward direction when a downward force F is applied to the leaf spring (indicated by reference numeral B).

The leaf spring member 12 can include a composite material with fiber in a resin matrix. For example, the leaf spring member 12 can be formed of carbon fibers, fiberglass, and the like, with a resin such as epoxy. The composite material can be shaped to form the arc of the one or more leaf spring(s) and can form a curvilinear spring member 12 that is flexible to store energy and resilient to return energy. An advantage with a plurality of thin carbon fiber leaf springs together forming the leaf spring member 12 is that the leaf spring member can be adapted to a particular individual wearer. This embodiment is not explicitly shown in any FIGS. 1-4, but in the claims.

As an example, the leaf spring member 12 can be embodied as a flat leaf spring member having width of ca. 20-56 mm and a thickness of 3-15 mm.

Typically, but not limited to, the lower limb prosthetic device 10 is adapted to provide translatory movement and the leaf spring member 12 is adapted to deflect also to some extent along a horizontal plane.

The stiffness of the leaf spring 12 may be varied according to the intended use. For example, some athletics use typically requires strength and thus incorporate a stiffer leaf spring, but other athletics use may require a softer leaf spring. In this way, a lower limb prosthetic device can provide a stiff or highly resilient response when a high load is applied, such as when the user runs, or a looser less resilient response when a lower load is applied, such as when the user walks.

In addition, the resilient characteristics of the energy storing feet can be altered in various ways to provide a more universal leaf spring which is capable of many different uses ranging from athletic use to more normal walking. For example, some leaf springs use multiple springs, bladders or resilient materials disposed between various elements, and/or multiple springs that deflect at different intervals of foot deflection in order to increase resistance as the force applied to the foot by the user increases.

While FIGS, 1-3 illustrate an exemplary configuration of a prosthetic foot (member) 30 primarily suitable for running or walking, and FIG. 4 illustrates an exemplary configuration of a prosthetic foot athletic member 30 primarily suitable for skiing 30, it is contemplated within the scope of the invention that the prosthetic foot member 30 may be of any suitable configuration and arrangement.

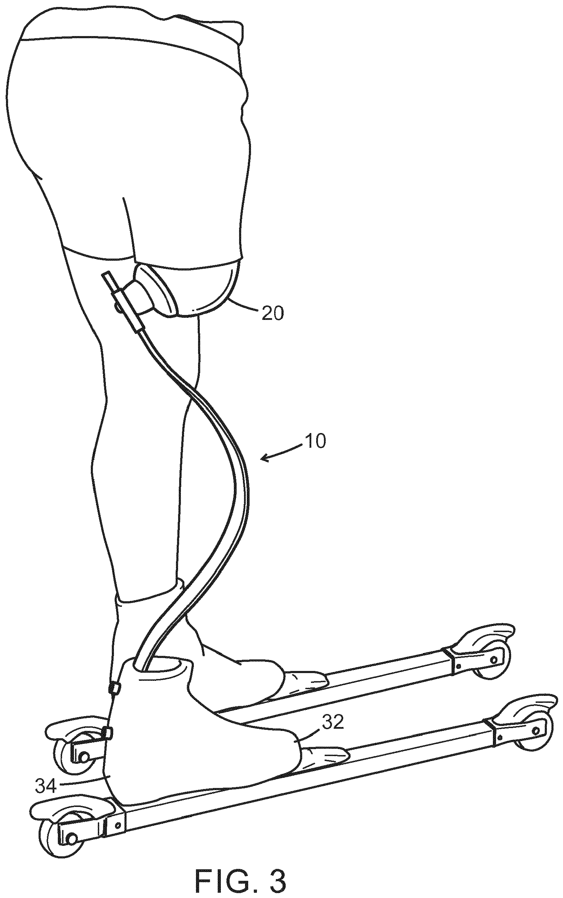

FIG. 3 is a perspective view of the limb prosthetic device 10 illustrated in use, herein skiing.

In FIG. 3, there is shown a perspective view of the lower limb prosthetic device 10 in an unloaded condition.

Referring now to FIG. 4, there is shown a perspective view of the lower limb prosthetic device 10 according to an alternative embodiment, wherein the leaf spring member 12 is C-formed. The leaf spring member 12 is connected to a straight elongate intermediate part 13, such as an adjustable tube, in the distal part 16. The intermediate part 13, which is adjustable in length, is then (adapted to be) further connected to the prosthetic foot member 30. By means of this embodiment, the length of the lower limb prosthetic device 10 can be adapted according to a desired length of a wear's leg for instance making the prosthetic foot more suited to different, including high impact, activities such as sprinting, running, cornering, hiking, and other athletic activities, or to fit different wearer's more easily since the intermediate part 13 can be adjusted according to different lengths.

In operation, several advantages of the embodiments of the lower limb prosthetic device are achieved. For example, the illustrated and disclosed embodiments of the lower limb prosthetic device are adapted to increase the resilient response. The lower limb prosthetic devices are also adapted to reduce the weight and size of the prosthetic device. The lower limb prosthetic devices are further adapted to improve the characteristics and roll over characteristics of the prosthetic device.

Although this description contains many specifics, these should not be construed as limiting the scope of the invention but as merely providing illustrations of some of the presently preferred embodiments thereof, as well as the best mode contemplated by the inventors of carrying out the invention. The invention, as described herein, is susceptible to various modifications and adaptations, and the same are intended to be comprehended within the meaning of the appended claims.

## Claims

1. A lower limb prosthetic device (10) adapted to be connected to an attachment section configured to attach to the limb, or a socket to receive the limb, of an amputee, the prosthetic device (10) comprising:
a leaf spring member (12), said leaf spring member (12) having a proximal, upper end (14), comprising an upper mounting device (14a), which can be attached to the attachment section, the leaf spring member (12) further comprising a distal, lower end (16), comprising a lower mounting device (16a), which can be attached to a prosthetic foot member, the leaf spring member (12) being connected to a straight elongate intermediate part (13), in the distal, lower end (16), wherein the straight elongate intermediate part (13) is adapted to be further connected to the prosthetic foot member;
**characterized in that**,
the straight elongate intermediate part (13) is adjustable in length and **in that** the upper mounting device (14a) and the lower mounting device (16a) are adapted to be adjustably attached by means of an upper releasable nut and bolt combination (14b), a lower releasable nut and bolt combination (16b), running in a respective upper groove (14b), and lower groove (16b), respectively.

2. The prosthetic device of claim 1, wherein the leaf spring member (12) has an original configuration and is adapted to be compressed when a load is applied to the lower limb prosthetic device (10) and return to the original configuration when the load is removed from the lower limb prosthetic device (10).

3. The prosthetic device of claim 1 or 2, wherein the leaf spring member (12) is adapted to deflect along a horizontal plane and a vertical plane.

4. The prosthetic device of claim 2, or 3, wherein the leaf spring member (12) comprises an arch and is resilient.

5. The prosthetic device of claim 4, wherein the arc is C-formed.

6. The prosthetic device of any one of the claims 1-5, wherein the leaf spring member (12) includes a composite material with fiber in a resin matrix.

7. The prosthetic device of claim 6, wherein leaf spring member (12) is formed of carbon fibers, fiberglass with a resin.

8. The prosthetic device of any one of the claims 3-7, wherein the leaf spring member (12) comprise a plurality of leaf springs.

## Patentansprüche

1. Prothesenvorrichtung (10) für untere Extremitäten, die dazu ausgelegt ist, mit einem Befestigungsabschnitt, der dazu konfiguriert ist, an der Extremität eines Amputierten befestigt zu werden, oder mit einer Pfanne, um die Extremität aufzunehmen, verbunden zu werden, wobei die Prothesenvorrichtung (10) Folgendes umfasst:
ein Blattfederelement (12), wobei das Blattfederelement (12) ein proximales, oberes Ende (14) aufweist, das eine obere Montagevorrichtung (14a) umfasst, die an dem Befestigungsabschnitt befestigt werden kann, wobei das Blattfederelement (12) ferner ein distales, unteres Ende (16) umfasst, das eine untere Montagevorrichtung (16a) umfasst, die an einem Prothesenfußelement befestigt werden kann, wobei das Blattfederelement (12) mit einem geraden länglichen Zwischenteil (13) in dem distalen, unteren Ende (16) verbunden ist, wobei der gerade längliche Zwischenteil (13) dazu ausgelegt ist, ferner mit dem Prothesenfußelement verbunden zu werden;
**dadurch gekennzeichnet, dass** die Länge des geraden länglichen Zwischenteils (13) anpassbar ist, und dadurch, dass die obere Montagevorrichtung (14a) und die untere Montagevorrichtung (16a) dazu ausgelegt sind, ferner mittels einer oberen lösbaren Kombination (14b) aus Mutter und Schraube, einer unteren lösbaren Kombination (16b) aus Mutter und Schraube, die in eine jeweilige obere Nut (14b) bzw. eine untere Nut (16b) verlaufen, verbunden zu werden.

2. Prothesenvorrichtung nach Anspruch 1, wobei das Blattfederelement (12) eine ursprüngliche Konfiguration aufweist und dazu ausgelegt ist, zusammengedrückt zu werden, wenn eine Last auf die Prothesenvorrichtung (10) für untere Extremitäten aufgebracht wird, und in die ursprüngliche Konfiguration zurückzukehren, wenn die Last von der Prothesenvorrichtung (10) für untere Extremitäten entfernt wird.

3. Prothesenvorrichtung nach Anspruch 1 oder 2, wobei das Blattfederelement (12) dazu ausgelegt ist, entlang einer horizontalen Ebene und einer vertikalen Ebene abgelenkt zu werden.

4. Prothesenvorrichtung nach Anspruch 2 oder 3, wobei das Blattfederelement (12) einen Bogen umfasst und elastisch ist.

5. Prothesenvorrichtung nach Anspruch 4, wobei der Bogen C-förmig ist.

6. Prothesenvorrichtung nach einem der Ansprüche 1-5, wobei das Blattfederelement (12) ein Verbundmaterial mit Fasern in einer Harzmatrix beinhaltet.

7. Prothesenvorrichtung nach Anspruch 6, wobei das Blattfederelement (12) aus Kohlefasern, Fiberglas mit einem Harz gebildet ist.

8. Prothesenvorrichtung nach einem der Ansprüche 3-7, wobei das Blattfederelement (12) eine Vielzahl von Blattfedern umfasst.

## Revendications

1. Dispositif prothétique (10) pour membre inférieur adapté pour être relié à une section de fixation configurée pour se fixer au membre, ou à un socle destiné à recevoir le membre, d'une personne amputée, le dispositif prothétique (10) comprenant :
un élément de ressorts à lames (12), ledit élément de ressorts à lames (12) ayant une extrémité supérieure proximale (14), comprenant un dispositif de montage supérieur (14a), qui peut être fixé à la section de fixation, l'élément de ressorts à lames (12) comprenant en outre une extrémité inférieure distale (16), comprenant un dispositif de montage inférieur (16a), qui peut être fixé à un élément de pied prothétique,
l'élément de ressorts à lames (12) étant relié à une partie intermédiaire rectiligne allongée (13), dans l'extrémité inférieure distale (16), dans lequel la partie intermédiaire rectiligne allongée (13) est adaptée pour être en outre reliée à l'élément de pied prothétique ; **caractérisé en ce que**,
la partie intermédiaire rectiligne allongée (13) est réglable en longueur et **en ce que** le dispositif de montage supérieur (14a) et le dispositif de montage inférieur (16a) sont adaptés pour être fixés de manière réglable au moyen d'une combinaison supérieure amovible d'écrou et boulon (14b), d'une combinaison inférieure amovible d'écrou et boulon (16b), s'étendant respectivement dans une rainure supérieure (14b) et une rainure inférieure (16b) respectives.

2. Dispositif prothétique selon la revendication 1, dans lequel l'élément de ressorts à lames (12) a une configuration d'origine et est adapté pour être comprimé lorsqu'une charge est appliquée au dispositif prothétique (10) pour membre inférieur et pour revenir à la configuration d'origine lorsque la charge est retirée du dispositif prothétique (10) pour membre inférieur.

3. Dispositif prothétique selon la revendication 1 ou 2, dans lequel l'élément de ressorts à lames (12) est adapté pour se fléchir le long d'un plan horizontal et d'un plan vertical.

4. Dispositif prothétique selon la revendication 2 ou 3, dans lequel l'élément de ressorts à lames (12) comprend un arc et est résilient.

5. Dispositif prothétique selon la revendication 4, dans lequel l'arc est en forme de C.

6. Dispositif prothétique selon l'une quelconque des revendications 1 à 5, dans lequel l'élément de ressorts à lames (12) comporte un matériau composite avec une fibre dans une matrice de résine.

7. Dispositif prothétique selon la revendication 6, dans lequel l'élément de ressorts à lames (12) est formé de fibres de carbone, fibre de verre avec une résine.

8. Dispositif prothétique selon l'une quelconque des revendications 3 à 7, dans lequel l'élément de ressorts à lames (12) comprend une pluralité de ressorts à lames.
